# EUROPÄISCHE PATENTSCHRIFT

(11) **EP 1 828 088 B1**
(45) Veröffentlichungstag und Bekanntmachung des Hinweises auf die Patenterteilung: **27.02.2008**
(21) Anmeldenummer: 05810883.8
(22) Anmeldetag: 21.11.2005
(51) Int. Cl.: C07C 29/74, B01D 53/00

(54) **VERFAHREN ZUR RÜCKGEWINNUNG VON METHANOL**
METHOD FOR THE RECOVERY OF METHANOL
PROCEDE POUR RECUPERER DU METHANOL

(30) Priorität: 20.12.2004 US 638365 P; 21.12.2004 DE 102004061352
(43) Veröffentlichungstag der Anmeldung: 05.09.2007
(73) Patentinhaber: Evonik Degussa GmbH, 40474 Düsseldorf (DE); Uhde GmbH, 44141 Dortmund (DE)
(72) Erfinder: HOFEN, Willi, 63517 Rodenbach (DE); HAAS, Thomas, 60322 Frankfurt (DE); KOLBE, Bärbel, 58452 Witten (DE)
(86) Internationale Anmeldenummer: PCT/EP2005/012418
(87) Internationale Veröffentlichungsnummer: WO 2006/066673

(56) Entgegenhaltungen:
- EP-A- 1 293 505
- EP-A- 1 424 332
- RU-C1- 2 159 664
- US-A- 4 659 866
- US-A1- 2003 040 637
- US-A1- 2003 144 535

## Beschreibung

Die Erfindung richtet sich auf ein verbessertes Verfahren zur Rückgewinnung von Methanol aus Gemischen, die Methanol und Wasser enthalten, das einen verringerten Energiebedarf für die Trennung hat.

Zur Herstellung von Propylenoxid kann Propylen mit Wasserstoffperoxid in Gegenwart eines titanhaltigen Zeolithkatalysators umgesetzt werden. In diesem Verfahren wird vorteilhaft Methanol als Lösungsmittel verwendet. Bei der Aufarbeitung der bei diesem Verfahren entstehenden Reaktionsmischung wird ein Gemisch erhalten, das Methanol und Wasser enthält. Aus diesem Gemisch muss Methanol zurückgewonnen werden, um es wieder in die Epoxidierungsreaktion zurückführen und das Verfahren wirtschaftlich betreiben zu können. Dabei muss das aus Wasserstoffperoxid entstehende Wasser, sowie gegebenenfalls mit wässrigem Wasserstoffperoxid in das Verfahren eingebrachtes Wasser aus dem Verfahren ausgeschleust werden. Die Rückgewinnung von Methanol aus dem Gemisch, das Methanol und Wasser enthält, verbraucht einen großen Teil der für das Verfahren aufzuwendenden Wärmeenergie. Für eine wirtschaftliche Durchführung des Verfahrens besteht deshalb ein Bedarf an einem verbesserten Verfahren zur Rückgewinnung von Methanol aus einem Gemisch, das Methanol und Wasser enthält, wobei das Verfahren einen verringerten Energiebedarf aufweist.

WO 02/02544 beschreibt ein Verfahren zur Herstellung von Propylenoxid, bei dem in der Aufarbeitung der Reaktionsmischung ein Gemisch erhalten wird, das Methanol, Wasser und geringe Mengen an Wasserstoffperoxid enthält. Das Dokument beschreibt die Abtrennung von Wasser aus diesem Gemisch durch eine in zwei Kolonnen durchgeführte Destillation, wobei die Drücke so gewählt werden, dass der Sumpf der Kolonne, die bei niedrigem Druck betrieben wird, mit den Brüden der zweiten Kolonne, die bei hohem Druck betrieben wird, beheizt wird. Mit der in WO 02/02544 beschriebenen Kopplung der Kolonnen lässt sich der Energiebedarf für die Rückgewinnung von Methanol aus der Mischung verringern.

Es besteht aber weiterhin ein Bedarf nach Verfahren zur Rückgewinnung von Methanol, die gegenüber dem Stand der Technik einen verringerten Energiebedarf haben. Diese Aufgabe wird durch das erfindungsgemäße Verfahren gelöst.

Gegenstand der Erfindung ist ein Verfahren zur Rückgewinnung von Methanol aus Gemischen, die Methanol und Wasser enthalten, wobei das Verfahren eine mehrstufige Eindampfung und eine nachgeschaltete Reihe aus Destillationsstufen umfasst und wobei das in der letzten Eindampfungsstufe der mehrstufigen Eindampfung erhaltene Sumpfprodukt der ersten Destillationsstufe der Reihe aus Destillationsstufen zugeführt wird. Die mehrstufige Eindampfung umfasst mindestens zwei Eindampfungsstufen, wobei jede Eindampfungsstufe einen Verdampfer umfasst und in der mehrstufigen Eindampfung der Druck von jeder Eindampfungsstufe zur nächsten Eindampfungsstufe reduziert wird. Der Verdampfer der zweiten Eindampfungsstufe und jeder nachfolgenden Eindampfungsstufe wird dabei mit dem dampfförmigen Kopfprodukt der jeweils vorangehenden Stufe beheizt. Die Reihe aus Destillationsstufen umfasst mindestens zwei Destillationsstufen, wobei jede Destillationsstufe eine Destillationskolonne und einen Verdampfer umfasst und wobei der zweiten und jeder nachfolgenden Destillationsstufe das Sumpfprodukt der jeweils vorangehenden Destillationsstufe zugeführt wird. In der Reihe der Destillationsstufen wird der Druck von jeder Destillationsstufe zur nächsten Destillationsstufe erhöht und mit Ausnahme der letzten Destillationsstufe wird in jeder Destillationsstufe der Verdampfer jeweils mit dem dampfförmigen Kopfprodukt der Destillationskolonne der nachfolgenden Destillationsstufe beheizt.

Vorzugsweise umfasst jede Eindampfungsstufe zusätzlich eine Destillationskolonne mit einem Verstärkungsteil, so dass in jeder Eindampfungsstufe ein Kopfprodukt mit einem Gehalt an Methanol von mehr als 90 Gew.-%, bevorzugt mehr als 95 Gew.-% erhalten wird.

Das erfindungsgemäße Verfahren umfasst eine mehrstufige Eindampfung mit mindestens zwei Eindampfungsstufen, wobei der Druck von jeder Eindampfungsstufe zur nächsten Eindampfungsstufe reduziert wird. Jede der Eindampfungsstufen umfasst einen Verdampfer, wobei für das erfindungsgemäße Verfahren alle Verdampfer eingesetzt werden können, die sich zur Verdampfung von Methanol aus Gemischen, die Methanol und Wasser enthalten, eignen. Vorzugsweise werden in den Eindampfungsstufen Durchlaufverdampfer eingesetzt, die ohne eine Umwälzpumpe betrieben werden können. In jeder Eindampfungsstufe wird dem Verdampfer das Gemisch flüssig zugeführt und durch die Verdampfung dampfförmige Brüden und ein flüssiges Sumpfprodukt erhalten. Das flüssige Sumpfprodukt wird jeweils dem Verdampfer der nächsten Eindampfungsstufe zugeführt. Wegen der Reduzierung des Drucks von jeder Eindampfungsstufe zur nächsten Eindampfungsstufe ist dazu in der Regel keine Pumpe erforderlich.

Der Verdampfer der ersten Eindampfungsstufe wird mit einem Heizmedium, vorzugsweise mit Dampf, beheizt. Der Verdampfer der zweiten Eindampfungsstufe und jeder nachfolgenden Eindampfungsstufe wird mit den Brüden, d.h. dem dampfförmigen Kopfprodukt, der jeweils vorangehenden Stufe beheizt.

Die Zahl der Eindampfungsstufen wird entsprechend der Zusammensetzung des Gemischs gewählt, aus dem Methanol zurückgewonnen werden soll und liegt vorzugsweise bei 3 bis 5 Eindampfungsstufen. Die Druckabstufung zwischen den Eindampfungsstufen wird so gewählt, dass der Siedepunkt des flüssigen Stroms, der der zweiten Eindampfungsstufe zugeführt wird, niedriger liegt als die Kondensationstemperatur der Brüden aus der vorangehenden Stufe, so dass die Kondensationswärme der Brüden zur Beheizung des Verdampfers genutzt wird und die Brüden in dem Verdampfer kondensiert werden. Das gleiche gilt auch für die Druckabstufung in den nachfolgenden Eindampfungsstufen. Die im letzten Verdampfer der mehrstufigen Eindampfung erhaltenen Brüden werden in einem separaten Kühler kondensiert, wobei vorzugsweise Kühlwasser zur Kondensation der Brüden verwendet wird.

Der Druck in der ersten Eindampfungsstufe wird vorzugsweise im Bereich von 3 bis 10 bar und besonders bevorzugt 4 bis 7 bar gewählt. Der Druck wird dabei so gewählt, dass ein ausreichendes Druck- und Temperaturgefälle über die Reihe der Eindampfungsstufen erreicht wird und zur Beheizung des ersten Verdampfers ein wirtschaftliches Heizmedium, vorzugsweise Dampf unter einem Druck von 4 bis 8 bar, verwendet werden kann. Der Druck in der letzten Verdampferstufe wird vorzugsweise im Bereich von 0,5 bis 2 bar und besonders bevorzugt 0,8 bis 1,5 bar gewählt. Der Druck wird so gewählt, dass über die Reihe der Eindampfungsstufe ein ausreichendes Druck- und Temperaturgefälle erreicht wird und die Brüden aus der letzten Eindampfungsstufe in wirtschaftlicher Weise mit Kühlwasser kondensiert werden können.

In einer bevorzugten Ausführungsform des erfindungsgemäßen Verfahrens weist jede Eindampfungsstufe zusätzlich eine Destillationskolonne mit einem Verstärkungsteil auf, so dass in jeder Eindampfungsstufe ein Kopfprodukt mit einem Gehalt an Methanol von mehr als 90 Gew.-% und vorzugsweise mehr als 95 Gew.-% erhalten wird. Der Destillationskolonne wird im unteren Teil der im Verdampfer erzeugte dampfförmige Strom zugeführt. Wahlweise kann zusammen mit dem dampfförmigen Strom auch das im Verdampfer resultierende flüssige Sumpfprodukt der Destillationskolonne zugeführt werden. Die Destillationskolonne kann als ein vom Verdampfer getrennter Apparat ausgeführt werden. Ebenso ist aber auch möglich, den Verdampfer und die dazu gehörige Destillationskolonne in einem Apparat auszubilden. Am Kopf der Kolonne werden die Brüden dampfförmig entnommen und dem Verdampfer der nachfolgenden Eindampfungsstufe zugeführt. Im oberen Teil der Kolonne wird zur Erzeugung eines Rücklaufs ein flüssiger Strom aus kondensierten Brüden eingespeist. Der zur Erzeugung des Rücklaufs verwendete Strom aus kondensierten Brüden kann dabei dem flüssigen Strom entnommen werden, der durch die Kondensation der Brüden in der nachfolgenden Eindampfungsstufe erhalten wird. Ebenso können aber auch die Brüden aller Eindampfungsstufen miteinander vereinigt werden und der flüssige Strom zur Erzeugung des Rücklaufs der Mischung der miteinander vereinigten Brüden entnommen werden. Der durch den Rücklauf im Sumpf der Kolonne erhaltene flüssige Strom wird zusammen mit dem Sumpfprodukt aus dem Verdampfer der nächsten Eindampfungsstufe zugeführt.

Für die bei dieser bevorzugten Ausführungsform in den Eindampfungsstufen verwendeten Destillationskolonnen wird die Zahl der Trennstufen und das Rücklaufverhältnis entsprechend der Zusammensetzung des im Verdampfer erzeugten dampfförmigen Stroms und der gewünschten Reinheit des zurückgewonnen Methanols gewählt. Das Rücklaufverhältnis ist dabei definiert als das Verhältnis der Masse des als Rücklauf flüssig eingespeisten Stroms zur Masse der dampfförmig am Kopf der Kolonne entnommenen Brüden. Die in den Eindampfungsstufen verwendeten Destillationskolonnen weisen vorzugsweise 2 bis 10 und besonders bevorzugt 3 bis 6 theoretische Trennstufen auf. Vorzugsweise werden in der Reihe der Eindampfungsstufen Kolonnen mit entlang der Reihe zunehmender Zahl an Trennstufen eingesetzt. Die in den Eindampfungsstufen verwendeten Destillationskolonnen können prinzipiell jede beliebige Bauform aufweisen und können beispielsweise als Bodenkolonnen oder als Packungskolonnen mit regelmäßigen oder unregelmäßigen Packungen ausgeführt werden. Die Destillationskolonnen in den Eindampfungsstufen werden vorzugsweise bei einem Rücklaufverhältnis im Bereich von 0,1 bis 1 und besonders bevorzugt 0,2 bis 0,6 betrieben. Vorzugsweise werden die Destillationskolonnen mit einem entlang der Reihe der Eindampfungsstufen zunehmenden Rücklaufverhältnis betrieben.

Das erfindungsgemäßen Verfahren umfasst weiterhin eine Reihe aus mindestens 2 Destillationsstufen, wobei in dieser Reihe der Druck von jeder Destillationsstufe zur nächsten Destillationsstufe erhöht wird. Jede der Destillationsstufen umfasst dabei eine Destillationskolonne und einen Verdampfer. Das in der letzten Eindampfungsstufe der mehrstufigen Eindampfung erhaltene flüssige Sumpfprodukt wird der ersten Destillationsstufe im mittleren Abschnitt der Destillationskolonne zugeführt. Der zweiten Destillationsstufe und jeder nachfolgenden Destillationsstufe wird jeweils das in der Destillationskolonne der vorangehenden Destillationsstufe erhaltene Sumpfprodukt in einem mittleren Abschnitt der Destillationskolonne zugeführt. In der letzten Destillationsstufe wird der Verdampfer mit einem Heizmedium, vorzugsweise mit Dampf beheizt. Die Verdampfer der vorangehenden Destillationsstufen werden jeweils mit den dampfförmigen Brüden der Destillationskolonne der unmittelbar nachfolgenden Destillationsstufe beheizt. Die am Kopf der Destillationskolonne der ersten Destillationsstufe erhaltenen Brüden werden in einem separaten Kühler kondensiert, wobei vorzugsweise Kühlwasser zur Kondensation der Brüden verwendet wird. Die Zahl der Destillationsstufen wird entsprechend der Zusammensetzung der Mischung, die in die erste Destillationsstufe eingespeist wird und dem gewünschten Restgehalt an Methanol im Sumpfprodukt, dass in der Destillationskolonne der letzten Destillationsstufe erhalten wird, gewählt und beträgt vorzugsweise zwei Destillationsstufen.

Die Druckabstufung in der Reihe aus Destillationsstufen wird so gewählt, dass der Siedepunkt im Sumpf der Destillationskolonne der ersten Destillationsstufe niedriger liegt als die Kondensationstemperatur der Brüden aus der Destillationskolonne der zweiten Destillationsstufe, so dass die Kondensationswärme der Brüden aus der zweiten Destillationsstufe zur Beheizung des Verdampfers der ersten Destillationsstufe benutzt wird und die Brüden in dem Verdampfer kondensiert werden. Das gleiche gilt auch für die Druckabstufung zwischen den weiteren Destillationsstufen.

In der ersten Destillationsstufe wird vorzugsweise ein Druck von 0,5 bis 2 bar und besonders bevorzugt 0,8 bis 1,5 bar verwendet, so dass ein ausreichendes Druck- und Temperaturgefälle über die Reihe der Destillationsstufen erreicht wird und die am Kopf der Kolonne der ersten Destillationsstufe anfallen Brüden auf wirtschaftliche Weise mit Kühlwasser kondensiert werden können. Der Druck in der letzten Destillationsstufe wird vorzugsweise im Bereich von 3 bis 10 bar, besonders bevorzugt 4 bis 7 bar gewählt, damit ein ausreichendes Druck- und Temperaturgefälle über die Reihe der Destillationsstufen erreicht wird und zur Beheizung des Verdampfers der letzten Destillationsstufe ein wirtschaftliches Heizmedium, vorzugsweise Dampf mit einem Druck von 4 bis 16 bar, verwendet werden kann.

Zu Erzeugung eines Rücklaufs wird bei jeder Destillationskolonne der Destillationsstufen am Kopf der Kolonne ein flüssiger Strom aus kondensierten Brüden eingespeist. Der flüssige Strom kann dabei den kondensierten Brüden entnommen werden, die durch Kondensation aus der gleichen Kolonne erhalten wurden. Ebenso können aber auch die in den Destillationskolonnen der Reihe aus Destillationsstufen erhaltenen Brüden miteinander vereinigt werden und der zur Erzeugung des Rücklaufs verwendete flüssige Strom der Mischung aus den vereinigten kondensierten Brüden entnommen werden.

Für die in der Reihe aus Destillationsstufen verwendeten Destillationskolonnen wird die Zahl der Trennstufen und das Rücklaufverhältnis vorzugsweise so gewählt, dass in jeder Kolonne ein Kopfprodukt mit einem Gehalt an Methanol von mehr als 90 Gew.-% und vorzugsweise mehr als 95 Gew.-% erhalten wird. Die in der Reihe von Destillationsstufen verwendeten Destillationskolonnen weisen vorzugsweise eine Trennwirkung von 10 bis 40 theoretischen Trennstufen auf. Prinzipiell sind alle Bauarten von Destillationskolonnen geeignet, beispielsweise Destillationskolonnen mit Böden oder Destillationskolonnen mit Packungen, wobei sowohl geordnete als auch ungeordnete Packungen verwendet werden können. Das Rücklaufverhältnis in den Kolonnen wird vorzugsweise so gewählt, dass es entlang der Reihe von Destillationsstufen zunimmt von einem Rücklaufverhältnis im Bereich von 0,3 bis 1 in der ersten Kolonne bis zu einem Rücklaufverhältnis im Bereich von 0,5 bis 3 in der letzten Kolonne.

Wenn das Methanol und Wasser enthaltende Gemisch, aus dem Methanol zurückgewonnen werden soll, noch Zwischensieder mit einem Siedepunkt zwischen den Siedepunkten von Methanol und Wasser enthält, kann die Destillationskolonne der letzten Destillationsstufe mit einem Seitenabzug versehen werden, aus dem ein Strom entnommen wird, in dem der Zwischensieder angereichert ist. Die Destillationskolonne der letzten Destillationsstufe kann für eine solche Ausführungsform auch vorteilhaft als Trennwandkolonne ausgeführt werden, um einen Strom mit einer hohen Konzentration an Zwischensieder zu erhalten.

Das erfindungsgemäße Verfahren eignet sich besonders zur Rückgewinnung von Methanol aus Gemischen, die Methanol in einer Konzentration von 70 Gew.-% und mehr enthalten. Das erfindungsgemäße Verfahren lässt sich vorteilhaft zur Rückgewinnung von Methanol aus Gemisch einsetzen, die bei der Aufarbeitung von Reaktionsmischungen anfallen, die in der Epoxidierung von Propylen mit Wasserstoffperoxid unter Verwendung von Methanol als Lösungsmittel anfallen. Solche Mischungen aus der Aufarbeitung der Reaktionsmischung einer Propylenepoxidierung enthalten typischerweise 70 bis 90 Gew.-% Methanol, bis zu 3 Gew.-% Zwischensieder, bis zu 3 Gew.-% Hochsieder und im Rest Wasser. Zwischensieder sind dabei alle Verbindungen, deren Siedepunkt zwischen den Siedepunkten von Methanol und Wasser liegt. Hochsieder sind dabei alle Verbindungen, deren Siedepunkt über dem Siedepunkt von Wasser liegt. Zur Auftrennung solcher Mischungen wird das erfindungsgemäße Verfahren vorzugsweise so ausgeführt, dass ausgehend von einer Mischung mit einem Methanolgehalt von 70 Gew.-% oder höher in der mehrstufigen Eindampfung eine Mischung mit einem Gehalt an Methanol im Bereich von 40 bis 60 Gew.-% erhalten wird und diese Mischung der Reihe von Destillationsstufen zugeführt wird. Die Betriebsbedingungen der Eindampfungsstufen und Destillationsstufen werden dabei jeweils so gewählt, dass Kopfprodukte mit einem Methanolgehalt von mehr als 90 Gew.-% und vorzugsweise mehr als 95 Gew.-% erhalten werden.

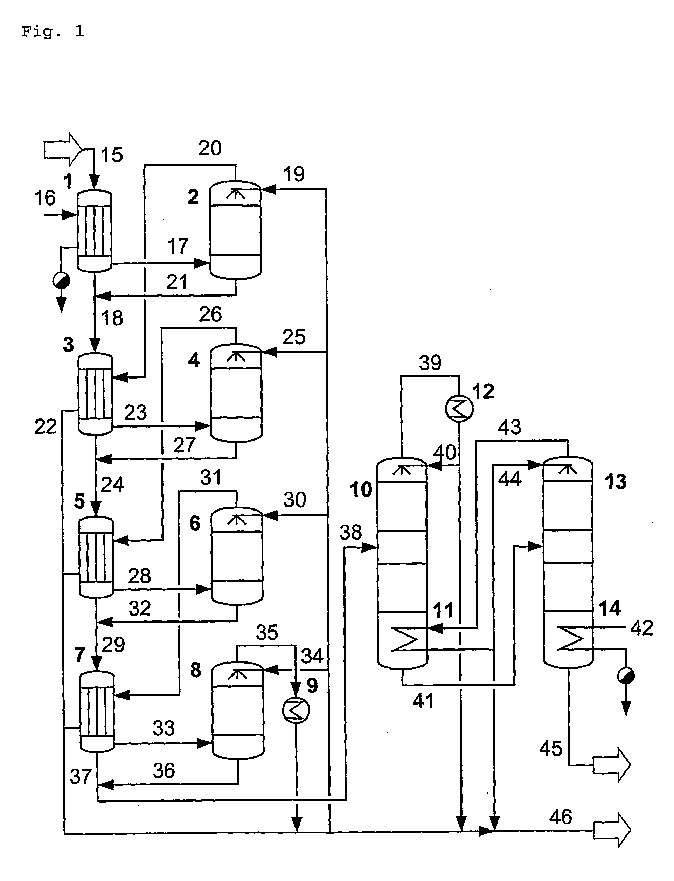

Figur 1 zeigt eine Ausführungsform des erfindungsgemäßen Verfahrens mit einer vierstufigen Eindampfung und einer nachfolgenden Reihe aus zwei Destillationsstufen. Die Eindampfungsstufen sind dabei mit einer jeweils getrennt ausgeführten Destillationsstufe versehen. In Figur 1 sind nur die im Verfahren benötigten Verdampfer, Destillationskolonnen und Kondensatoren dargestellt. Für die Ausführung des Verfahrens noch erforderliche Pumpen, Zwischenbehälter und Armaturen sind der Übersichtlichkeit halber nicht wiedergegeben und können von einem Fachmann entsprechnd seinem Fachwissen ergänzt werden.

In der am meisten bevorzugten Ausführungsform wird das erfindungsgemäße Verfahren in einer Anordnung entsprechend Figur 1 ausgeführt.

Das Verfahren umfasst dabei eine vierstufige Eindampfung, wobei jede Eindampfungsstufe einen im Durchlauf betriebenen Verdampfer und eine Destillationskolonne mit einem Verstärkungsteil umfasst. Die erste Eindampfungsstufe mit dem Verdampfer (1) und der Destillationskolonne (2), die einen Verstärkungsteil mit 3 theoretischen Trennstufen aufweist, wird bei einem Druck von 5 bar betrieben. Die zweite Eindampfungsstufe mit dem Verdampfer (3) und der Destillationskolonne (4), die einen Verstärkungsteil mit 3 theoretischen Trennstufen aufweist, wird bei einem Druck von 2,8 bar betrieben. Die dritte Eindampfungsstufe mit dem Verdampfer (5) und der Destillationskolonne (6), die einen Verstärkungsteil mit 4 theoretischen Trennstufen aufweist, wird bei einem Druck von 1,8 bar betrieben. Die vierte und letzte Eindampfungsstufe mit dem Verdampfer (7) und der Destillationskolonne (8) mit einem Verstärkungsteil mit 6 theoretischen Trennstufen wird bei einem Druck von 1,0 bar betrieben. Die vierte Eindampfungsstufe umfasst zusätzlich einen Brüdenkondensator (9).

Der vierstufigen Eindampfung nachgeschaltet ist eine Reihe aus zwei Destillationsstufen. Die erste Destillationsstufe umfasst die Destillationskolonne (10) mit einer Trennwirkung von 18 theoretischen Trennstufen, einem in die Kolonne integrierten Verdampfer (11) und einem Brüdenkondensator (12) und wird bei einem Druck von 1 bar betrieben. Die zweite Destillationsstufe umfasst die Destillationskolonne (13) mit einer Trennleistung von 17 theoretischen Trennstufen und dem in die Kolonne integrierten Verdampfer (14) und wird bei einem Druck von 4 bar betrieben.

Dem Verdampfer (1) der ersten Eindampfungsstufe wird der Strom (15) zugeführt, aus dem Methanol zurück gewonnen werden soll und der einen Gehalt an 75 bis 85 Gew.-% Methanol, bis zu 3 Gew.-% Zwischensiedern, bis zu 1 Gew.-% Hochsiedern und im Rest Wasser aufweist. Der Verdampfer (1) wird über den Strom (16) mit Dampf unter einem Druck von 4 bar beheizt. Die dem Verdampfer (1) zugeführte Dampfmenge wird so gewählt, dass etwa 25 Gew.-% des zugeführten Stromes (15) verdampft und als Strom (17) dampfförmig der Destillationskolonne zugeführt werden. Das im Verdampfer mit einer Temperatur von ca. 118 °C anfallende flüssige Sumpfprodukt wird als Strom (18) dem Verdampfer der zweiten Eindampfungsstufe zugeführt. Am Kopf der Destillationskolonne (2) wird über Strom (19) Brüdenkondensat flüssig aufgegeben, so dass ein Rücklaufverhältnis von 0,2 resultiert. Die am Kopf der Destillationskolonne (2) erhaltenen dampfförmigen Brüden werden als Strom (20) dem Verdampfer (3) der zweiten Eindampfungsstufe als Heizmedium zugeführt. Der im Sumpf der Destillationskolonne anfallende flüssige Strom (21) wird zusammen mit dem Strom (18) dem Verdampfer der zweiten Eindampfungsstufe zugeführt.

In der zweiten Eindampfungsstufe werden im Verdampfer (3) die dampfförmigen Brüden der ersten Eindampfungsstufe kondensiert und als flüssiges Brüdenkondensat (22) erhalten. Der damit verdampfte Strom (23) wird der Destillationskolonne (4) zugeführt. Das im Verdampfer erhaltene flüssige Sumpfprodukt (24) wird mit einer Temperatur von ca. 100 °C dem Verdampfer (5) der dritten Verdampfungsstufe zugeführt. Am Kopf der Destillationskolonne 4 wird zur Erzeugung des Rücklaufs über Strom (25) flüssiges Brüdenkondensat aufgegeben, so dass sich ein Rücklaufverhältnis von 0,25 ergibt. Die am Kopf der Kolonne 4 erhaltenen dampfförmigen Brüden werden mit Strom (26) dem Verdampfer (5) der dritten Eindampfungsstufe als Heizmedium zugeführt. Das in der Kolonne (4) im Sumpf anfallende flüssige Produkt wird als Strom (27) zusammen mit Strom (24) dem Verdampfer (5) der dritten Eindampfungsstufe zugeführt.

Die im Verdampfer der zweiten Eindampfungsstufe erhaltenen dampfförmigen Brüden werden im Verdampfer (5) der dritten Eindampfungsstufe kondensiert und das flüssige Kondensat mit dem Strom (22) vereinigt. Der in Verdampfer (5) erzeugte dampfförmige Strom (28) wird der Destillationskolonne (6) zugeführt. Das flüssige Sumpfprodukt des Verdampfers (5) wird mit einer Temperatur von ca. 87 °C dem Verdampfer (7) der vierten und letzten Eindampfungsstufe zugeführt. Am Kopf der Destillationskolonne (6) wird über Strom (30) flüssiges Brüdenkondensat aufgegeben, so dass sich ein Rücklaufverhältnis von 0,35 ergibt. Die am Kopf der Kolonne (6) anfallen dampfförmigen Brüden werden über Strom (31) dem Verdampfer (7) der vierten Eindampfungsstufe als Heizmedium zugeführt. Das im Sumpf der Kolonne (6) erhalten flüssige Produkt wird als Strom (32) zusammen mit Strom (29) dem Verdampfer der vierten Eindampfungsstufe zugeführt.

Im Verdampfer (7) der vierten Eindampfungsstufe werden die dampfförmigen Brüden (31) aus der dritten Eindampfungsstufe kondensiert und das Kondensat mit dem Strom (22) vereinigt. Der im Verdampfer (7) verdampfte Produktstrom (33) wird der Destillationskolonne (8) zugeführt. Am Kopf der Destillationskolonne (8) wird als Strom (34) flüssiges Brüdenkondensat aufgegeben, so dass ein Rücklaufverhältnis von 0,4 resultiert. Die am Kopf der Kolonne (8) erhaltenen dampfförmigen Brüden werden als Strom (35) im Kondensator (9) kondensiert und das flüssige Kondensat mit dem Strom (22) vereinigt. Das im Sumpf der Kolonne (8) erhaltene flüssige Produkt (36) wird mit dem flüssigen Sumpfprodukt des Verdampfers (7) vereinigt und als Strom (38) der ersten Destillationsstufe zugeführt.

Der flüssige Strom (38), der die mehrstufige Eindampfung verlässt, enthält noch ca. 45 bis 50 Gew.-% Methanol und wird der Destillationskolonne (10) der ersten Destillationsstufe zugeführt. Die am Kopf der Destillationskolonne (10) erhaltenen dampfförmigen Brüden (39) werden im Kondensator (12) kondensiert und ein Teil des erhalten Brüdenkondensats wird mit Strom (40) als Rücklauf am Kopf der Kolonne (10) aufgegeben, so dass ein Rücklaufverhältnis von 0,4 resultiert. Der restliche Teil der kondensierten Brüden wird mit Strom (22) vereinigt. Das in der Destillationskolonne (10) erhaltene flüssige Sumpfprodukt (41) wird der Destillationskolonne (13) der zweiten Destillationsstufe zugeführt. Der Verdampfer (14) der zweiten Destillationsstufe wird über Strom (42) mit Dampf unter einem Druck von 8 bar beheizt, so dass sich eine Sumpftemperatur von ca. 143 °C einstellt. Die am Kopf der Destillationskolonne (13) erhaltenen dampfförmigen Brüden werden als Strom (43) dem Verdampfer (11) der ersten Destillationsstufe als Heizmedium zugeführt. Von den im Verdampfer (11) kondensierten Brüden wird ein Teil als Strom (44) als Rücklauf auf den Kopf der Kolonne (13) zurückgeführt, so dass ein Rücklaufverhältnis von 1,1 resultiert. Der verbleibende Anteil der kondensierten Brüden wird mit Strom (22) vereinigt. Im Sumpf der Kolonne (13) wird ein Strom (45) erhalten, der nur noch weniger als 0,5 Gew.-% Methanol enthält. Die im Verfahren anfallenden Brüdenströme (20), (26), (31), (35), (39) und (43) werden nach Kondensation vereinigt und liefern einen Produktstrom (46), der mehr als 90 Gew.-% Methanol und weniger als 4 Gew.-% Wasser enthält.

In dieser Ausführungsform beträgt der Energiebedarf für die Rückgewinnung von Methanol ca. 157 kWh/t Methanol. Für die Rückgewinnung von Methanol in einer einzelnen Kolonne ohne Wärmeintegration sind dagegen ca. 447 kWh/t Methanol erforderlich. Bei dem aus WO 02/02544 bekannten Verfahren mit zwei Kolonnen mit Wärmeintegration liegt der Energiebedarf bei ca. 246 kWh/t Methanol. Durch das erfindungsgemäße Verfahren mit einer Wärmeintegration sowohl in der mehrstufigen Eindampfung als auch in der nachfolgenden Reihe aus Destillationsstufen lässt sich gegenüber einer Methanolrückgewinnung ohne Wärmeintegration somit etwa 65 % der benötigten Heizenergie einsparen. Gegenüber dem aus WO 02/02544 bekannten Verfahren ergibt sich eine Energieeinsparung von etwa 36 %.

## Patentansprüche

1. Verfahren zur Rückgewinnung von Methanol aus Gemischen, die Methanol und Wasser enthalten, umfassend
a) eine mehrstufige Eindampfung mit mindestens zwei Eindampfungsstufen, wobei jede Eindampfungsstufe einen Verdampfer umfasst und in der mehrstufigen Eindampfung der Druck von jeder Eindampfungsstufe zur nächsten Eindampfungsstufe reduziert wird und der Verdampfer der zweiten Eindampfungsstufe und jeder nachfolgenden Eindampfungsstufe mit dem dampfförmigen Kopfprodukt der jeweils vorangehenden Stufe beheizt wird, sowie
b) eine Reihe aus mindestens zwei Destillationsstufen,
wobei jede Destillationsstufe eine Destillationskolonne und einen Verdampfer umfasst und wobei der zweiten und jeder nachfolgenden Destillationsstufe das Sumpfprodukt der jeweils vorangehenden Destillationsstufe zugeführt wird, in der Reihe der Destillationsstufen der Druck von jeder Destillationsstufe zur nächsten Destillationsstufe erhöht wird und mit Ausnahme der letzten Destillationsstufe in jeder Destillationsstufe der Verdampfer jeweils mit dem dampfförmigen Kopfprodukt der Destillationskolonne der nachfolgenden Destillationsstufe beheizt wird,
wobei das in der letzten Eindampfungsstufe der mehrstufigen Eindampfung a) erhaltene Sumpfprodukt der ersten Destillationsstufe der Reihe aus Destillationsstufen b) zugeführt wird.

2. Verfahren nach Anspruch 1,
**dadurch gekennzeichnet**
**dass** die mehrstufige Eindampfung 3 bis 5 Eindampfungsstufen umfasst.

3. Verfahren nach Anspruch 1 oder 2,
**dadurch gekennzeichnet**
**dass** der Druck in der ersten Eindampfungsstufe im Bereich von 3 bis 10 bar, vorzugsweise 4 bis 7 bar und der Druck in der letzten Eindampfungsstufe im Bereich von 0,5 bis 2 bar, vorzugsweise 0,8 bis 1,5 bar liegt.

4. Verfahren nach einem der vorangehenden Ansprüche,
**dadurch gekennzeichnet**
**dass** jede Eindampfungsstufe zusätzlich eine Destillationskolonne mit einem Verstärkungsteil umfasst, so dass in jeder Eindampfungsstufe ein Kopfprodukt mit einem Gehalt an Methanol von mehr als 90 Gew.-%, bevorzugt mehr als 95 Gew.-% erhalten wird.

5. Verfahren nach Anspruch 4,
**dadurch gekennzeichnet**
**dass** der Verstärkungsteil jeweils eine Trennleistung von 2 bis 10 theoretischen Böden aufweist und mit einem Rücklaufverhältnis im Bereich von 0,1 bis 1 betrieben wird.

6. Verfahren nach Anspruch 5,
**dadurch gekennzeichnet**
**dass** in jeder Eindampfungsstufe der Verstärkungsteil eine Trennleistung von 3 bis 6 theoretischen Böden aufweist.

7. Verfahren nach Anspruch 5 oder 6,
**dadurch gekennzeichnet**
**dass** in jeder Eindampfungsstufe der Verstärkungsteil mit einem Rücklaufverhältnis im Bereich von 0,2 bis 0,6 betrieben wird.

8. Verfahren nach einem der vorangehenden Ansprüche,
**dadurch gekennzeichnet,**
**dass** die Reihe aus Destillationsstufen zwei Destillationsstufen umfasst.

## Claims

1. Process for recovering methanol from mixtures containing methanol and water, comprising
a) multistage evaporation with at least two evaporation stages, wherein each evaporation stage comprises an evaporator and in the multistage evaporation the pressure is reduced from each evaporation stage to the next and the evaporator of the second evaporation stage and each subsequent evaporation stage is heated with the vaporous overhead of the in each case preceding stage, and
b) a series of at least two distillation stages, wherein each distillation stage comprises a distillation column and an evaporator and wherein the bottoms of the in each case preceding distillation stage are supplied to the second and each subsequent distillation stage, the pressure is increased in the series of distillation stages from each distillation stage to the next and, with the exception of the final distillation stage, in each distillation stage the evaporator is heated in each case by the vaporous overhead of the distillation column of the subsequent distillation stage,
wherein the bottoms obtained in the final evaporation stage of the multistage evaporation a) are supplied to the first distillation stage of the series of distillation stages b).

2. Process according to claim 1,
**characterized in that**
the multistage evaporation comprises 3 to 5 evaporation stages.

3. Process according to claim 1 or claim 2,
**characterized in that**
the pressure in the first evaporation stage is in the range from 3 to 10 bar, preferably 4 to 7 bar and the pressure in the final evaporation stage is in the range from 0.5 to 2 bar, preferably 0.8 to 1.5 bar.

4. Process according to any one of the preceding claims,
**characterized in that**
each evaporation stage additionally comprises a distillation column with a rectifying section, such that an overhead with a methanol content of more than 90 wt.%, preferably more than 95 wt.%, is obtained in each evaporation stage.

5. Process according to claim 4,
**characterized in that**
the rectifying section exhibits in each case a separation effect of 2 to 10 theoretical plates and is operated with a reflux ratio in the range from 0.1 to 1.

6. Process according to claim 5,
**characterized in that**
in each evaporation stage the rectifying section exhibits a separation effect of 3 to 6 theoretical plates.

7. Process according to claim 5 or claim 6,
**characterized in that**
in each evaporation stage the rectifying section is operated with a reflux ratio in the range from 0.2 to 0.6.

8. Process according to any one of the preceding claims,
**characterized in that**
the series of distillation stages comprises two distillation stages.

## Revendications

1. Procédé de récupération de méthanol hors de mélanges qui contiennent du méthanol et de l'eau, comprenant
a) une concentration par évaporation à plusieurs étapes avec au moins deux étapes de concentration par évaporation, chaque étape de concentration par évaporation comprenant un évaporateur et, dans la concentration par évaporation à plusieurs étapes, la pression est réduite à chaque fois d'une étape de concentration par évaporation à la suivante et l'évaporateur de la deuxième étape de concentration par évaporation et de chaque étape de concentration par évaporation suivante étant chauffé à l'aide du produit de tête sous forme de vapeur de l'étape à chaque fois précédente, et
b) une série d'au moins deux étapes de distillation, chaque étape de distillation comprenant une colonne de distillation et un évaporateur et le produit de puits de l'étape de distillation à chaque fois précédente étant acheminé à chaque étape de distillation suivante, dans la série d'étapes de distillation, la pression étant augmentée à chaque d'une étape de distillation à la suivante et, à l'exception de la dernière étape de distillation, dans chaque étape de distillation, l'évaporateur étant chauffé à chaque fois à l'aide du produit de tête sous forme de vapeur de la colonne de distillation de l'étape de distillation suivante,
le produit de puits obtenu dans la dernière étape de concentration par évaporation de la concentration par évaporation à plusieurs étapes a) étant acheminé à la première étape de distillation de la série des étapes de distillation b).

2. Procédé selon la revendication 1, **caractérisé en ce que** la concentration par évaporation à plusieurs étapes comprend de 3 à 5 étapes de concentration par évaporation.

3. Procédé selon la revendication 1 ou 2, **caractérisé en ce que** la pression dans la première étape de concentration par évaporation se situe dans le domaine de 3 à 10 bars, de préférence, de 4 à 7 bars et **en ce que** la pression dans la dernière étape de concentration par évaporation se situe dans le domaine de 0,5 à 2 bars, de préférence, de 0,8 à 1,5 bar.

4. Procédé selon l'une quelconque des revendications précédentes, **caractérisé en ce que** chaque étape de concentration par évaporation comprend en outre une colonne de distillation ayant une partie de renforcement, de telle sorte que, dans chaque étape de concentration par évaporation, l'on obtienne un produit de tête ayant une teneur en méthanol de plus de 90 % en poids, de préférence, de plus de 95 % en poids.

5. Procédé selon la revendication 4, **caractérisé en ce que** la partie de renforcement présente à chaque fois un effet de séparation de 2 à 10 plateaux théoriques et est opérée avec un taux de reflux dans le domaine de 0,1 à 1.

6. Procédé selon la revendication 5, **caractérisé en ce que**, dans chaque étape de concentration par évaporation, la partie de renforcement présente un effet de séparation de 3 à 6 plateaux théoriques.

7. Procédé selon la revendication 5 ou 6, **caractérisé en ce que**, dans chaque étape de concentration par évaporation, la partie de renforcement est opérée avec un taux de reflux de 0,2 à 0,6.

8. Procédé selon l'une quelconque des revendications précédentes, **caractérisé en ce que** la série d'étapes de distillation comprend deux étapes de distillation.
